# EUROPÄISCHE PATENTANMELDUNG

(11) **EP 1 258 571 A2**
(43) Veröffentlichungstag der Anmeldung: **20.11.2002**
(21) Anmeldenummer: 02009713.5
(22) Anmeldetag: 30.04.2002
(51) Int. Cl.: E03D 9/03

(54) **Abgabevorrichtung**

(30) Priorität: 14.05.2001 DE 10124554
(71) Anmelder: Buck-Chemie GmbH ., 71083 Herrenberg (DE)
(72) Erfinder: Dettinger, Johannes, Dr., 72160 Horb am Neckar (DE); Jaeschke, Edgar, Dipl.-Ing., 70794 Filderstadt (DE); Seidel, Detlef, Dipl.-Ing., 71131 Jettingen (DE)
(74) Vertreter: Patentanwälte Bartels und Partner

(57) **Zusammenfassung**

2. Die Erfindung betrifft eine Abgabevorrichtung für ein Sanitärmittel zum Reinigen und/oder Desinfizieren und/oder zur Duftstoffabgabe, vorzugsweise für den Einsatz in Sanitärgegenständen, wie Toiletten-Spülbecken, mit einem der Aufnahme des Sanitärmittels dienenden Behälter (10), der hierfür einen mit einem Gehäuse (12) begrenzten Aufnahmeraum (14) aufweist. Dadurch das der Aufnahmeraum (14) von einer von Hand betätigbaren Austrageinrichtung (16) durchgriffen ist, die einen Austragkolben (18) aufweist, der das Sanitärmittel aus dem Aufnahmeraum (14) des Behälters (10) in Richtung einer Abgabeöffnung (20) des Behälters (10) schiebt, um dergestalt das Sanitärmittel auf den Sanitärgegenstand aufzutragen, ist eine Abgabevorrichtung für Sanitärmittel derart weiterentwickelt, dass für eine Bedienperson die Berührung mit Teilen der Sanitärgegenstandes vermieden ist, wobei auch hochviskose Sanitärmittel zum Einsatz kommen können.

## Beschreibung

Die Erfindung betrifft eine Abgabevorrichtung für ein Sanitärmittel zum Reinigen und/oder Desinfizieren und/oder zur Duftstoffabgabe, vorzugsweise für den Einsatz in Sanitärgegenständen, wie Toiletten-Spülbecken, mit einem der Aufnahme des Sanitärmittels dienenden Behälter, der hierfür einen mit einem Gehäuse begrenzten Aufnahmeraum aufweist.

Durch die EP 0 940 509 A2 sind Abgabevorrichtungen für Toilettenbecken bekannt mit einem aus zwei Schalenhälften bestehenden Aufnahmebehälter für eine wasserlösliche, hochviskose Wirkstoffzubereitung, insbesondere in Form eines Gels oder einer Paste, mit Eintrittsöffnungen für das Spülwasser und Austrittsöffnungen für die vom Spülwasser jeweils angelösten Teile der Wirkstoffzubereitung, wobei die Austrittsöffnungen derart im Aufnahmebehälter angeordnet und voneinander getrennt sind, daß bei abnehmender Wirkstoffmenge und absinkendem Spülwasserniveau im Aufnahmebehälter die angelösten Teile der Wirkstoffzubereitung über eine der Austrittsöffnungen in das Toilettenbecken abgebbar sind. Die Größe der Austrittsöffnungen ist dabei derart bemessen, daß sie für wässrige Flüssigkeiten mit Viskositäten bis zu 3000 mPas durchlässig sind, nicht jedoch für die Wirkstoffzubereitung mit Viskositäten über 3000 mPas. Die genannten Austrittsöffnungen verlaufen entlang einer bogenförmigen Trennlinie im Aufnahmebehälter und sind durch Abstandsstege voneinander getrennt. Sofern man bei der bekannten Lösung die Austrittsöffnungen in alternierender Reihenfolge mit den Abstandsstegen in unterschiedlichen Nivenauebenen anordnet, besteht unabhängig vom Spülwasserniveau im Aufnahmebehälter immer mindestens eine Austrittsmöglichkeit über eine der Austrittsöffnungen für die über das Spülwasser jeweils angelöste Wirkstoffzubereitung. Hierdurch ist gewährleistet, daß die Wirkstoffzubereitung im Aufnahmebehälter mit entsprechend langen Standzeiten bevorratet werden kann und dennoch bei jedem Spülvorgang eine dosierte Abgabe des angelösten Wirkstoffmittels fortwährend über eine der zugeordneten Auslaßöffnungen erfolgt.

Eine dahingehende Abgabevorrichtung verfügt über einen Einhängebügel, an dem der Behälter mit der Wirkstoffzubereitung festgelegt ist und über den sich der Behälter am Rand der Toilettenschüssel festlegen läßt.

Diese bekannte Abgabevorrichtung ist für die Abgabe von gelartigen Sanitärmitteln geeignet, bestehend im wesentlichen aus tensidhaltigen Pasten und Gelen, die mit handelsüblichen Verdickern hergestellt werden. Nachteilig bei dieser bekannten Lösung ist, daß zum Nachfüllen der Abgabevorrichtung mit Sanitärmittel häufig diese aus der Toilette für den Nachfüllvorgang.entnommen und gegebenenfalls von Verunreinigungen und Gelresten befreit werden muß, bevor sie nachgefüllt wieder für eine gewisse Einsatzdauer zur Verfügung steht. Insbesondere bei häufigen Spülanwendungen kommt es darüber hinaus zu einem raschen Austrag des gelartigen Reinigungsmittels, was häufiger entsprechende Nachfüllvorgänge notwendig macht. Sofern bei den modernen Abgabesystemen ein Nachfüllen des Sanitärmittels über Nachfüll- und Abgabeflaschen erfolgt, mit denen es möglich ist, den in der Toilette befindlichen Abgabebehälter über eine hierfür vorgesehene Nachfüllöffnung nachträglich zu befüllen, ohne daß der Behälter von Hand aus der Toilette entnommen werden muß, kommt neben dem Abgabebehälter in der Toilette noch ein Nachfüllbehältnis zum Einsatz, was die Gesamtkosten für dahingehende Gesamtsysteme erhöht. Auch hat es sich in der Praxis gezeigt, daß trotz der komfortabel durchzuführenden Nachbefüllung die Abgabevorrichtung zumindest zeitweise immer noch von Hand angefaßt werden muß, beispielsweise, um diese für den Nachfüllvorgang am Toilettenrand festzuhalten, so daß insoweit Berührungen nicht zu vermeiden sind. Im Zuge der Weiterentwicklung von Sanitärmitteln wäre es auch wünschenswert, Reinigungsmedien mit hochviskosen Eigenschaften, die im Wertebereich deutlich über 3000 mPas liegen, für Reinigungsvorgänge im Toilettenbecken einzusetzen. Dies ist mit den bekannten Lösungen nicht möglich.

Ausgehend von diesem Stand der Technik liegt der Erfindung die Aufgabe zugrunde, unter Beibehalten der Vorteile der bekannten Abgabevorrichtungen für Sanitärmittel diese derart weiter zu entwickeln, daß für eine Bedienperson die Berührung mit Teilen des Sanitärgegenstandes vermieden ist, daß die Kosten für eine dahingehende Abgabevorrichtung reduziert sind und daß auch hochviskose Sanitärmittel zum Einsatz kommen können. Eine dahingehende Aufgabe löst eine Abgabevorrichtung mit den Merkmalen des Patentanspruches 1 in seiner Gesamtheit.

Dadurch, daß gemäß dem kennzeichnenden Teil des Patentanspruches 1 der Aufnahmeraum von einer von Hand betätigbaren Austrageinrichtung durchgriffen ist, die einen Austragkolben aufweist, der das Sanitärmittel aus dem Aufnahmeraum des Behälters in Richtung einer Abgabeöffnung des Behälters schiebt, um dergestalt das Sanitärmittel von Hand auf den Sanitärgegenstand aufzutragen, kann auf die konventionellen Abgabevorrichtungen mit am Toilettenbecken festzulegenden Behältern ebenso verzichtet werden wie auf entsprechende Nachfüllsysteme in Form von Nachfüllbehältnissen, wie Flaschen od. dgl..

Mit der erfindungsgemäßen Abgabevorrichtung läßt sich über die von Hand betätigbare Austrageinrichtung das Medium in den Sanitärgegenstand, wie ein Toilettenspülbecken, in einer Aufstreichbewegung an die Beckenwand abgeben, ohne daß die Bedienperson mit Teilen des Beckens oder sonstigen Teilen eines Sanitärgegenstandes in Berührung kommt. Da das Sanitärmittel über die Abgabevorrichtung des Behälters abgegeben wird, kommt darüber hinaus bei sachgerechtem Gebrauch die Abgabevorrichtung gleichfalls nicht in Berührung mit dem Sanitärgegenstand, so daß auch insoweit gesteigerten hygienischen Vorschriften Rechnung getragen wird. Über die Austrageinrichtung mit Austragkolben ist darüber hinaus gewährleistet, daß immer eine definitiv vorgebbare Sanitärmittelmenge in das Becken des Sanitärgegenstandes abgegeben wird. Vorzugsweise erfolgt dabei die Abgabe an Beckenteilen, die nicht vom stehenden Wasser in der Toilettenschüssel überdeckt sind, sofern es sich um eine üblich aufgebaute Toilette handelt. Ferner hat es sich gezeigt, daß mit der erfindungsgemäßen Abgabevorrichtung auch ausgesprochen hochviskose Sanitärmittel abgegeben werden können, was mit den üblichen Abgabevorrichtungen nicht erreichbar ist. Auf diese Art und Weise läßt sich in einer größeren Varianz eine Wirkstoffzubereitung zusammenstellen und als hochwirksames Sanitärmittel in.ein Toiletten-Spülbecken od. dgl. abgeben.

Bei einer bevorzugten Ausführungsform der erfindungsgemäßen Abgabevorrichtung weist das mit ihr abzugebende Sanitärmittel einen Haftvermittler auf, wobei der Haftvermittler aus der Gruppe der länger- oder langkettigen organischen Moleküle, die wenigstens teilweise hydrophil sind, ausgewählt ist und der hydrophile Teil des Haftvermittlers wechselwirkt bei Anwesenheit von Wasser wenigstens teilweise mit den Wassermolekülen und wird dabei derart klebrig, daß das Mittel in Anwesenheit geringer Mengen an Wasser unmittelbar auf den Sanitärgegenstand appliziert werden kann und dort dergestalt haftet, daß das Mittel erst nach einer größeren Anzahl von Spülvorgängen vollständig abspülbar ist. Ein dahingehend haftendes Sanitärmittel, das sich sehr gut für die Abgabe mit der vorstehend beschriebenen Abgabevorrichtung eignet, ist in der WO 99/66017 bereits vorgestellt worden.

Bei einer weiteren bevorzugten Ausführungsform der erfindungsgemäßen Abgabevorrichtung verfügt die Austrageinrichtung über einen Spindelkörper, längs dessen der Austragkolben verfahrbar ist, der über das Gehäuse des Behälters außenumfangsseitig geführt mittig über eine Gewindeführung in das Außengewinde des Spindelkörpers eingreift. Vorzugsweise ist dabei des weiteren vorgesehen, daß der Spindelkörper an seinem einen freien Ende einen Anschlag für den Austragkolben aufweist oder der dahingehende Anschlag ist durch das Gehäuseende gebildet, das die Abgabeöffnung des Behälters umfaßt. Über den dahingehenden Spindelantrieb läßt sich zielgerichtet die Austragung des Sanitärmittels aus dem Behälter vornehmen und in Abhängigkeit der zum Einsatz kommenden Gewindegänge für die Gewindeführung ist eine dosierte genau abgestufte Abgabe des Sanitärmittels möglich.

Bei einer besonders bevorzugten Ausführungsform der erfindungsgemäßen Abgabevorrichtung ist die Behälteröffnung mit einer Auftrageinrichtung versehen, insbesondere in Form einer Dosierhilfe. Vorzugsweise weist dabei die Dosierhilfe einen länglichen Abgabeschlitz auf, der einen flächigen Auftrag des Sanitärmittels auf den jeweiligen Sanitärgegenstand erlaubt. Über den dahingehenden düsenartigen Abgabeschlitz ist der kontinuierliche Auftrag des Sanitärmittels in das Toliletten-Spülbecken erreichbar und es ist über eine größere Breite der Flächenauftrag mit dem Sanitärmittel möglich. Auf diese Art und Weise steht eine größere, später bespülbare Fläche zur Verfügung, so daß über die schlitzartige Dosierhilfe sichergestellt ist, daß genügend Wirkstoffmedium für die anstehende Reinigung und/oder Desinfizierung und/oder Beduftung zur Verfügung steht.

Sofern vorzugsweise die Dosierhilfe außenumfangsseitig von einem Abschlußteil als Teil eines Deckelteils umgreifbar ist, läßt sich über das Abschlußteil ein hermetischer Abschluß des Behälterinhalts erreichen, so daß ungewollt eine Duftstoffabgabe vermieden ist und im übrigen ist das im Behälter noch bevorratete Sanitärmittel von schädlichen Umgebungseinflüssen verläßlich abgeschlossen.

Bei einer weiteren bevorzugten Ausführungsform der erfindungsgemäßen Abgabevorrichtung sind die Querschnitte der Außenumfänge von Behälter, Austragkolben, Dosierhilfe und Deckelteil oval gehalten, so daß sich in platzsparender Weise und darüber hinaus auch formschön das Sanitärmittel sich in der Abgabevorrichtung bevorraten läßt.

Bei einer Art einer bevorzugten Ausführungsform der erfindungsgemäßen Abgabevorrichtung ist vorgesehen, daß der Spindelkörper an seinem anderen freien Ende mit einer Betätigungseinrichtung zusammenwirkt, die dem Drehantrieb des Spindelkörpers dient. Vorzugsweise weist dabei die Betätigungseinrichtung ein Drehrad auf, das im Gehäuse des Behälters drehbar gelagert von Gehäuseteilen teilweise derart freigehalten ist, daß eine Zugriffsmöglichkeit für die Betätigung von Hand besteht. Neben dem angesprochenen Drehrad läßt sich der Spindelkörper jedoch auch über von Hand betätigbare Klinken und Rasten betätigen. Hierbei können von Hand betätigbare Klinken am Gehäuse bewegbar angebracht in Zahnräder am Außenumfang des Drehteils angeordnet eingreifen, um dergestalt das Drehteil in einer Richtung für den Spindelantrieb weiterzudrehen. Entsprechend in die Zähne eingreifende Rasten stellen dann sicher, daß es nicht ungewollt zu einer rückdrehenden Bewegung des Drehteils kommt, bei der gegebenenfalls der Auftragkolben in der zur Behälteröffnung entgegengesetzten Richtung verfahren würde, mit der Folge, daß es dann zu Problemen bei der Abgabe des Sanitärmittels käme.

Im folgenden wird die erfindungsgemäße Abgabevorrichtung anhand der Zeichnung näher erläutert. Dabei zeigen in prinzipieller und nicht maßstäblicher Darstellung die
- Fig. 1: in Ansicht einen Längsschnitt durch die erfindungsgemäße Abgabevorrichtung;

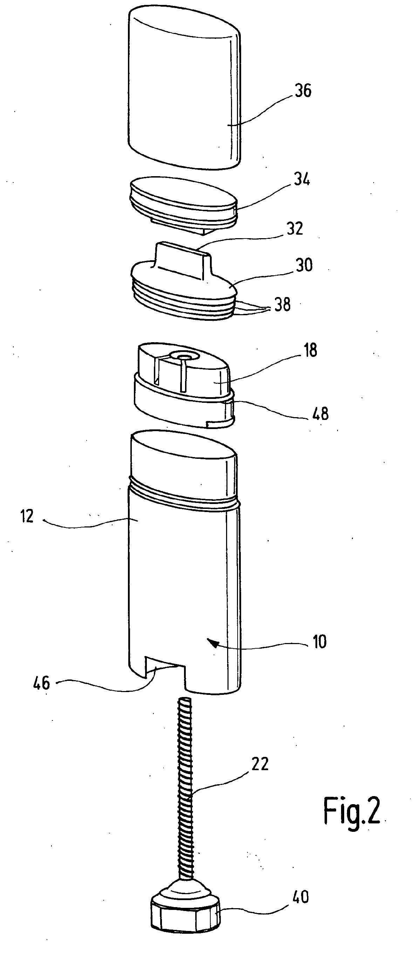

- Fig. 2: in der Art einer technischen Explosionszeichnung die wesentlichen Einzelkomponenten der Abgabevorrichtung.

Die erfindungsgemäße Abgabevorrichtung nach den Figuren dient der Abgabe eines nicht näher dargestellten Sanitärmittels in einen Sanitärgegenstand, wie beispielsweise in ein Toiletten-Spülbecken (nicht dargestellt). Das Sanitärmittel selbst dient dem Reinigen und/oder Desinfizieren und/oder der Duftstoffabgabe. Für die Aufnahme des Sanitärmittels ist ein als Ganzes mit 10 bezeichneter Behälter vorgesehen, der hierfür einen mit einem Gehäuse 12 begrenzten Aufnahmeraum 14 aufweist. Der Aufnahmeraum 14 selbst ist von einer von Hand betätigbaren, als Ganzes mit 16 bezeichneten Austrageinrichtung durchgriffen, die einen Austragkolben 18 aufweist. Mittels der Austrageinrichtung 16 und des Austragkolbens 18 ist es möglich, das nicht näher dargestellte Sanitärmittel aus dem Aufnahmeraum 14 des Behälters 10 in Richtung einer Abgabeöffnung 20 des Behälters 10 zu schieben und dergestalt das Sanitärmittel in das nicht näher dargestellte Toilettenbecken aufzutragen. Vorzugsweise werden dabei diejenigen inneren Wandteile des Toilettenbeckens bestrichen, die bei einem üblichen Toilettenaufbau keinen Rest an Spülwasser bevorraten.

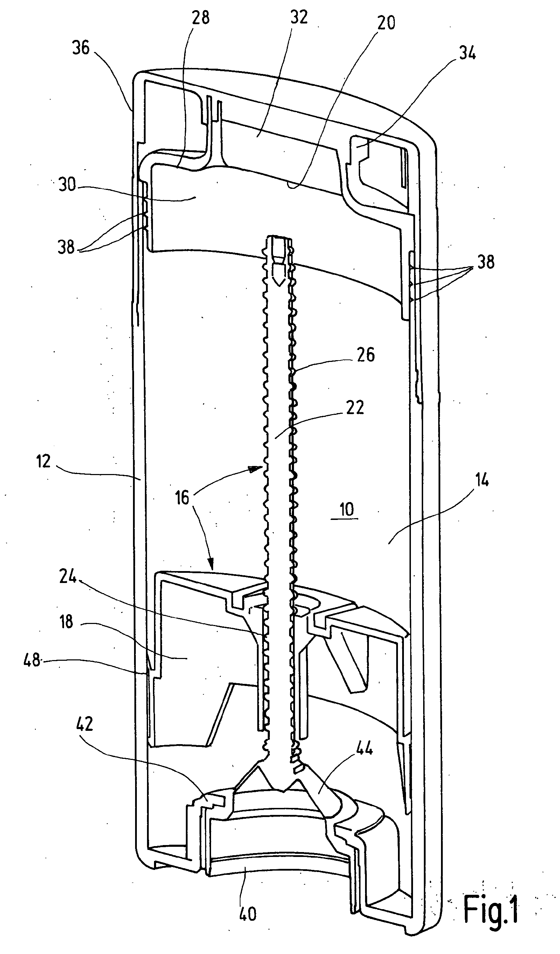

Die genannte Austrageinrichtung 16 verfügt über einen Spindelkörper 22, längs dessen der Austragkolben 18 verfahrbar gehalten ist. Der Austragkolben 18 ist über das Gehäuse 12 des Behälters 10 außenumfangsseitig geführt. Des weiteren weist der Austragkolben 18 mittig eine.Gewindeführung 24 auf, in die das Außengewinde 26 des Spindelkörpers 22 eingreift. Wie insbesondere die Fig.1 zeigt, bildet.also der Spindelkörper 22 eine Art Spindelstange mit Außengewinde aus, wobei bei einer drehenden Bewegung des Spindelkörpers 22 entlang seiner Längsachse die Gewindeführung 24 mitgeführt wird und in Abhängigkeit der Drehrichtung wird der Austragkolben 18 in Richtung auf die Fig.1 gesehen in Richtung der Abgabeöffnung 20 bewegt oder in der entgegengesetzten Richtung. .

Der Spindelkörper 22 kann an seinem einen freien Ende einen Anschlag für den Austragkolben 18 aufweisen, um dergestalt die Längsverfahrbarkeit für den Auftragkolben 18 zu begrenzen. In der vorgestellten Ausführungsform jedoch ist der dahingehende Anschlag 28 durch das Gehäuseende gebildet, das die Abgabeöffnung 20 des Behälters 10 umfaßt. Zur Bildung des dahingehenden Anschlages 28 ist das vordere Ende des Gehäuses 12 nach innen umgekröpft, wobei das Gehäuse 12 in der tatsächlichen Ausführungsform nach der Fig.1 auch mehrteilig ausgebildet sein kann, so daß das Gehäuseende selbst durch ein anderes Behälterteil gebildet wird.

Das dahingehend separate Abschlußteil ist aus einer Auftrageinrichtung 30 gebildet, insbesondere in Form einer Dosierhilfe für die Abgabe des Sanitärmittels. Hierfür weist die Dosierhilfe einen länglichen Abgabeschlitz 32 auf, der einen flächigen Auftrag des Sanitärmittels auf den jeweiligen Sanitärgegenstand erlaubt. Über den Abgabeschlitz 32 läßt sich in einer breiten Bahn und in einer streichenden Bewegung das Sanitärmittel auf den Bekkeninnenrand auftragen. Die Dosierhilfe wiederum in Form der Auftrageinrichtung 30 ist außenumfangsseitig insbesondere im Bereich des nach vorne sich erstreckenden Abgabeschlitzes 32 von einem Abschlußteil 34 als Teil eines Deckelteils 36 umgreifbar. Dadurch ist es möglich, im wesentlichen hermetisch das Innere des Behälters 10 gegenüber der Umgebung abzudichten, so daß Geruchsstoffe nicht ungewollt aus dem Behälter 10 nach außen treten können. Um eine sichere Abdichtung zwischen Auftrageinrichtung 30 und Deckelteil 36 herstellen zu können, umgreift das Abschußteil 34 mit einer vorgebbaren Vorspannkraft elastisch den vorgelagerten Bereich der Auftrageinrichtung 30 mit dem Abgabeschlitz 32. Ferner kann es möglich sein, daß das Abschlußteil 34 mit einer entsprechenden Vorkragung auch in das Innere des Abgabeschlitzes 32 eingreift, um derart eine Dichtwirkung sicherzustellen.

Die Auftrageinrichtung 30 kann mit dem freien Ende des Behälters 10 über ein nicht näher dargestelltes Gewindesystem verschraubt werden, sofern die genannten Bauteile zylindrisch ausgebildet sind; im vorliegenden Fall bei ovaler Ausgestaltung ist ein Einschraubvorgang jedoch nicht möglich, so daß die Auftrageinrichtung 30 über mehrere, hintereinander angeordnete Längsrippen 38 mit der Behälteröffnung verklippt wird. Das Abschlußteil 34 wiederum kann einstückiger Bestandteil des Deckelteils 36 sein; es besteht aber auch hier die Möglichkeit, die genannten Bauteile über eine Klippverbindung od. dgl. miteinander zu verbinden.

Die Querschnitte der Außenumfänge von Behälter 10, Austragkolben 18, Auftrageinrichtung 30 und Deckelteil 36 sind oval ausgebildet. Hierdurch ergibt sich ein platzsparender Aufbau der Gesamtabgabevorrichtung, wobei die ovale Anordnung derart gewählt ist, daß die breiteste Stelle des Abgabebehälters 10 parallel zum Abgabelängsschlitz 32 verläuft, um dergestalt in der vorgesehenen Auftragrichtung einen klein aufbauenden Behälter 10 zu erhalten, der dennoch von seine Aufnahmekapazität her für das Sanitärmittel optimiert ist.

Wie des weiteren die Figuren zeigen, ist der Spindelkörper 22 an seinem anderen freien Ende mit einer Betätigungseinrichtung versehen, die dem Drehantrieb des Spindelkörpers 22 dient. Hierfür weist die Betätigungseinrichtung ein von Hand betätigbares Drehrad 40 auf, das im Gehäuse 12 des Behälters 10 drehbar gelagert von Gehäuseteilen auf der Vorder- und Rückseite derart freigehalten ist, daß eine Zugriffsmöglichkeit für eine Bedienperson besteht. Für die Aufnahme des Drehrades 40 weist demgemäß im unteren Bereich des Behälters 10 diese eine nach innen umgebördelte Lagerstelle 42 auf, die das Drehrad 40 derart umfaßt, daß es in der Längsausrichtung des Spindelkörpers 22 frei drehbar ist. Das Drehrad 40 kann innen hohl ausgebildet sein, um derart eine Gewichtsersparnis zu erreichen sowie seine Fertigung zu erleichtern. An seiner in Blickrichtung auf die Figuren gesehen oberen Seite weist das Drehrad 40 einen Aufnahmekonus 44 auf für die Aufnahme des unteren Endes des Spindelkörpers 22. Hierfür ist der Aufnahmekonus 44, wie dies die Fig. 1 zeigt, nochmals nach innen umgebördelt. Des weiteren ergibt sich aus der Fig. 1, daß das freie untere Ende des Drehrades 40 mit der Unterseite der Lagerstelle 42 im wesentlichen bündig abschließt und damit mit dem Gehäuseboden des Behälters 10. Für den Antrieb des Drehrades 40 von Hand ist an der Unterseite des Behälters 10 zumindest eine freigehaltene Stelle 46 vorgesehen, die von dem Drehrad 40 teilweise entlang seines Außenumfanges durchgriffen wird.

An der Unterseite des Austragkolbens 18 ist dieser.als Hohlkörper ausgebildet, so daß er in platzsparender Weise zumindest teilweise die umgebördelte Lagerstelle 42 des Gehäuses 12 umfassen kann. Hierdurch wird die freie Volumenmenge im Aufnahmeraum 14 erhöht. Ferner ist der Austragkolben 18 außenumfangsseitig mit einer Dichtleiste 48 umgeben, um zu vermeiden, daß ungewollt Sanitärmittel aus dem Aufnahmeraum 14 in Richtung der Lagerstelle 42 und mithin aus dem Behälter 10 an seine Unterseite gelangen kann. Alle Komponenten lassen sich somit in einfacher Weise zusammenstecken und derart montieren, wobei sich ein Teil der Verbindungsstellen auch über eine Klebstoffverbindung in sinnfälliger Weise erreichen läßt.

Der Austragkolben 18 ist auf seiner Oberseite im wesentlichen eben ausgebildet und bildet dergestalt eine Anlagefläche für das auszuschiebende Sanitärmittel aus. Betätigt man nun das Drehrad 40 im Uhrzeigersinn, folgt der Spindelkörper 22 dieser Bewegung nach und über die Gewindeführung 24 wird der Austragkolben 18 in Richtung der Abgabeöffnung 20 bewegt. Es besteht die Möglichkeit, die in dem Abgabeschlitz 32 befindliche Sanitärmittelmenge abzugeben oder die dahingehende Abgabebewegung durch Betätigen des Vorschubmechanismusses zu unterstützen, sofern die Bedienperson das Drehrad 40 fortlaufend bei der Abgabe betätigt. Die Vorschubeinrichtung über ein Drehrad 40 zu betätigen, läßt sich kostengünstig realisieren. Es besteht aber bei nicht näher dargestellten Ausführungsformen. auch die Möglichkeit, die Drehbewegung des Spindelkörpers 22 über eine Klinkenansteuerung oder einen Ratschenmechanismus zu veranlassen.

Die Abgabe über den Abgabeschlitz 32 erfolgt selbstredend bei abgenommenem Deckelteil 36, wobei die dahingehende Abgabestelle über das Deckelteil 36 und das Abschlußteil 34 dann wieder hermetisch verschlossen werden kann. Die bereits angesprochene freigehaltene Stelle 46 kann zur besseren Betätigung des Drehrades 40 auch noch auf der Rückseite des Behälters 10 in gleicher Höhe vorhanden sein. Ferner bleibt das Drehrad 40 im Bereich der Lagerstelle 42 in axialer Richtung fixiert, sofern sich der Austragkolben 18 über seine Dichtleiste 48 außenumfangsseitig am Inneren des Gehäuses 12 abstützen kann und im übrigen entlang der Gewindeführung 24 mittig längs des Spindelkörpers 22 geführt und gehalten ist. Zusätzliche Fixierungsmittel können, müssen demgemäß aber nicht vorhanden sein.

Vorzugsweise bestehen alle wesentlichen Komponenten der Abgabevorrichtung aus einem üblichen Kunststoffmaterial, das sich insbesondere spritzgießtechnisch gut verarbeiten läßt. Ferner sind alle Komponenten, sofern möglich, als Hohlkammerteile ausgebildet, um dergestalt eine Gewichtsersparnis zu erreichen. Des weiteren kann die Abgabevorrichtung aus recycelfähigem Kunststoffmaterial bestehen; es besteht gegebenenfalls aber auch die Möglichkeit, dahingehende Abgabevorrichtungen im Werk nachzubefüllen und/oder aber in Bereichen der täglichen Anwendung dahingehender Abgabevorrichtungen. Die Abgabevorrichtung läßt sich hygienisch betätigen und bedienen, da ein Hautkontakt zwischen Bedienperson und Toilettenbecken nicht zustande kommt, da dies durch die Abgabevorrichtung selbst verhindert wird. Ist die Abgabeöffnung hinreichend mit Sanitärmittel gefüllt, ist im übrigen eine Einhandbetätigung möglich, bei der die Bedienperson von außen her den Behälter 10 mit seinem Gehäuse 12 umfaßt, wobei das Deckelteil 36 abgenommen ist.

Als Sanitärmittel für den Einsatz in der vorstehend beschriebenen Abgabevorrichtung sind insbesondere solche geeignet, wie sie in der PCT - Anmeldung WO 99/66017 veröffentlicht sind. Geeignete Sanitärmittel zum Reinigen und/oder Desinfizieren und/oder zur Duftstoffabgabe umfassen dabei einen Haftvermittler, Wasser, anionische und/oder nicht-ionische und/oder amphotere Tenside sowie gegebenenfalls weitere übliche Bestandteile, wie Duftstoffe, Verdicker, Farbstoffe und Konservierungsstoffe. Der Haftvermittler wird vorzugsweise aus der Gruppe der länger- oder langkettigen organischen Moleküle, die wenigstens teilweise hydrophil sind, ausgewählt, wobei der hydrophile Teil des Haftvermittlers bei Anwe- senheit von Wasser wenigstens teilweise mit den Wassermolekülen wechselwirkt und klebrig wird, so daß das Mittel in Anwesenheit geringer Mengen an Wasser unmittelbar auf den Sanitärgegenstand appliziert werden kann und dort haftet. Die Viskosität des Mittels soll wenigstens 15000 mPas betragen, so daß das Mittel erst nach einer größeren Anzahl von Spülvorgängen vollständig aus dem Toilettenspülbecken abspülbar ist. Die genannte Viskosität wurde mit einem HAAKE - Viskosimeter bestimmt, wobei als Sanitärmittel auch sog. rigide Gele eingesetzt werden können mit höheren Viskositäten um 50000 mPas, besonders bevorzugt mit mehr-als 150000 mPas. Im Hinblick auf die sehr hohen Viskositäten ist.es jedenfalls vorteilhaft, die Abgabe über einen Abgabeschlitz 32 vorzunehmen in der Art einer Auftragsdüse, angeordnet im Bereich der freien Abgabeöffnung des Behälters 10.

## Patentansprüche

1. Abgabevorrichtung für ein Sanitärmittel zum Reinigen und/oder Desinfizieren und/oder zur Duftstoffabgabe, vorzugsweise für den Einsatz in Sanitärgegenständen, wie Toiletten-Spülbecken, mit einem der Aufnahme des Sanitärmittels dienenden Behälter (10), der hierfür einen mit einem Gehäuse (12) begrenzten Aufnahmeraum (14) aufweist, **dadurch gekennzeichnet, daß** der Aufnahmeraum (14) von einer von Hand betätigbaren Austrageinrichtung (16) durchgriffen ist, die einen Austragkolben (18) aufweist, der das Sanitärmittel aus dem Aufnahmeraum (14) des Behälters (10) in Richtung einer Abgabeöffnung (20) des Behälters (10) schiebt; um dergestalt das Sanitärmittel auf den Sanitärgegenstand aufzutragen.

2. Abgabevorrichtung nach Anspruch 1, **dadurch gekennzeichnet, daß** das mit ihr abzugebende Sanitärmittel einen Haftvermittler aufweist, wobei der Haftvermittler aus der Gruppe der länger- oder langkettigen organischen Moleküle, die wenigstens teilweise hydrophil sind, ausgewählt ist und der hydrophile Teil des Haftvermittlers bei Anwesenheit von Wasser wenigstens teilweise mit den Wassermolekülen wechselwirkt und dabei derart klebrig wird, daß das Mittel in Anwesenheit geringer Mengen an Wasser unmittelbar auf den Sanitärgegenstand appliziert werden kann und dort dergestalt haftet, daß das Mittel erst nach einer größeren Anzahl von Spülvorgängen vollständig abspülbar ist.

3. Abgabevorrichtung nach Anspruch 1 oder 2, **dadurch gekennzeichnet, daß** die Austrageinrichtung (16) über einen Spindelkörper (22) verfügt, längs dessen der Austragkolben (18) verfahrbar ist, der über das Gehäuse (12) des Behälters (10) außenumfangsseitig geführt mittig über eine Gewindeführung (24) in das Außengewinde des Spindelkörpers (22) eingreift.

4. Abgabevorrichtung nach Anspruch 3, **dadurch gekennzeichnet, daß** der Spindelkörper (22) an seinem einen freien Ende einen Anschlag für den Austragkolben (18) aufweist oder der dahingehende Anschlag (28) ist durch das Gehäuseende gebildet, das die Abgabeöffnung (20) des Behälters (10) umfaßt.

5. Abgabevorrichtung nach Anspruch 4, **dadurch gekennzeichnet, daß** die Behälteröffnung mit einer Auftrageinrichtung (30), insbesondere in Form einer Dosierhilfe versehen ist.

6. Abgabevorrichtung nach Anspruch 5, **dadurch gekennzeichnet, daß** die Dosierhilfe einen länglichen Abgabeschlitz (32) begrenzt, der einen flächigen Auftrag des Sanitärmittels auf den jeweiligen Sanitärgegenstand erlaubt.

7. Abgabevorrichtung nach Anspruch 6, **dadurch gekennzeichnet, daß** die Dosierhilfe außenumfangsseitig von einem Abschlußteil (34) als Teil eines Deckelteils (36) umgreifbar ist.

8. Abgabevorrichtung nach Anspruch 7, **dadurch gekennzeichnet, daß** die Querschnitte der Außenumfänge von Behälter (10), Austragkolben (18), Auftrageinrichtung (30) und Deckelteil (36) oval sind.

9. Abgabevorrichtung nach einem der Ansprüche 4 bis 8, **dadurch gekennzeichnet, daß** der Spindelkörper (22) an seinem anderen freien. Ende mit einer Betätigungseinrichtung zusammenwirkt, die dem Drehantrieb des Spindelkörpers (22) dient.

10. Abgabevorrichtung nach Anspruch 9, **dadurch gekennzeichnet, daß** die Betätigungseinrichtung ein Drehrad (40) aufweist, das im Gehäuse (12) des Behälters (10) drehbar gelagert von Gehäuseteilen teilweise derart freigehalten ist, daß eine Zugriffsmöglichkeit für die Betätigung von Hand besteht.
